# EUROPÄISCHE PATENTSCHRIFT

(11) **EP 2 406 205 B1**
(45) Veröffentlichungstag und Bekanntmachung des Hinweises auf die Patenterteilung: **30.11.2016**
(21) Anmeldenummer: 10707227.4
(22) Anmeldetag: 05.03.2010
(51) Int. Cl.: C07C 17/093, C07C 25/02

(54) **VERFAHREN ZUR HERSTELLUNG VON CHLOR- UND BROMAROMATEN**
METHOD FOR MANUFACTURING CHLORINE AND BROMINE AROMATIC COMPOUNDS
PROCÉDÉ DE FABRICATION D'AROMATES DE CHLORE ET DE BROME

(30) Priorität: 12.03.2009 EP 09155017
(43) Veröffentlichungstag der Anmeldung: 18.01.2012
(73) Patentinhaber: Bayer Intellectual Property GmbH, 40789 Monheim am Rhein (DE)
(72) Erfinder: HIMMLER, Thomas, 51519 Odenthal (DE)
(74) Vertreter: BIP Patents
(86) Internationale Anmeldenummer: PCT/EP2010/001395
(87) Internationale Veröffentlichungsnummer: WO 2010/102761

(56) Entgegenhaltungen:
- EP-A1- 0 458 006
- WO-A-00/78712
- FR-A- 2 475 535
- TRAUGOTT SANDMEYER: "Ueber die Ersetzung der Amidgruppe durch Chlor, Brom und Cyan in den aromatischen Substanzen" CHEMISCHE BERICHTE, Bd. 17, Nr. 2, 1884, Seiten 2650-2653, XP002540175

## Beschreibung

Die vorliegende Erfindung betrifft ein neues Verfahren zur Herstellung von Chlor- und Bromaromaten. Solche Chlor- und Bromaromaten dienen unter anderem als Ausgangsmaterialien zur Herstellung von 2-Aryl-malonsäure-dinitrilen (Synlett 2006, 3167-9; WO 2004/050607) und Phenylessigsäuren (siehe beispielsweise Tetrahedron Letters 45 (2004) 4261-4). Solche 2-Aryl-malonsäure-dinitrile und Phenylessigsäuren wiederum sind wichtige Zwischenprodukte zur Herstellung von Verbindungen mit akarizider (siehe beispielsweise DE -A-4216814), insektizider (siehe beispielsweise WO 98/5638) oder herbizider (siehe beispielsweise WO 04/80962; WO 99/47525; WO 2000/78881) Wirkung.

Die Herstellung von Chlor- und Bromaromaten kann durch verschiedene, seit langem bekannte Methoden erfolgen. Dies sind beispielsweise die direkte Chlorierung oder Bromierung der entsprechenden aromatischen Verbindungen, oder die Überführung eines Anilins in die mit Chlor oder Brom substituierte aromatische Verbindung durch eine Sandmeyer-Reaktion (Houben-Weyl Bd. V/3, S. 846ff.; Houben-Weyl Bd. V/4, S. 437ff.).

FR 2 475 535 offenbart ein Verfahren zur Herstellung von Chloraromaten.

Die Sandmeyer-Reaktion zur Herstellung eines Chlor- oder Bromaromaten aus dem entsprechenden Anilin wird in der Regel so durchgeführt, dass man das Anilin in wässriger Lösung oder Suspension in Gegenwart von Salz- oder Bromwasserstoffsäure mittels Zugabe von Natrium- oder Kaliumnitrit diazotiert, und anschließend durch Zusatz eines Kupfer(I)-salzes (CuCl; CuBr) die Einführung des Chlor- oder Bromatoms unter Abspaltung von Stickstoff bewirkt. Dabei kann das Kupfersalz prinzipiell auch in unterstöchiometrischer Menge eingesetzt werden. Dies führt jedoch in machen Fällen zu unbefriedigenden Ausbeuten. Es sind auch Methoden bekannt geworden, diese Reaktion ohne Zusatz eines Metallsalzes durchzuführen (WO 2000/78712, Bsp. P1). Ebenfalls ist bekannt, diese Reaktion so durchzuführen, dass man die Diazotierung ohne Verwendung von Wasser mittels eines Alkylnitrits in einem organischen Lösungsmittel durchführt. Anschließend kann man nach verschiedenen Methoden entweder mit stöchiometrischen Mengen eines Kupfersalzes umsetzen; unterstöchiometrische Mengen eines Kupfersalzes und HCl- oder HBr-Gas einsetzen; oder in speziellen Fällen ganz auf den Zusatz eines Übergangsmetallsalzes verzichten (WO 2006/084663).

Da Alkylnitrite eine nur begrenzte thermische Stabilität besitzen, ist aus technischer Sicht die Diazotierung mittels Natrium- oder Kaliumnitrit vorzuziehen.

Die Verwendung der Kupfersalze zur Umsetzung der Diazoniumverbindungen in die Chlor- oder Bromaromaten wird zwar seit langer Zeit technisch realisiert, hat aber den Nachteil, dass die kupferhaltigen Abfälle entsorgt werden müssen. Das Kupfer(I)-salz wird dabei in der Regel in stöchiometrischen Mengen eingesetzt. Versucht man die Menge kupferhaltiger Abfälle dadurch zu verringern, dass man das Kupfer(I)chlorid oder -bromid in einem unterstöchiometrischen Anteil einsetzt, kommt es bei der Reaktion häufig zu vermehrter Bildung von unerwünschten Nebenprodukten wie beispielsweise der entsprechenden Phenole durch Verkochung des Diazoniumsalzes. Es ist deshalb auch bereits versucht worden, andere Metallsalze als Kupfersalze zur Durchführung der Sandmeyer-Reaktion einzusetzen. So ist beispielsweise bekannt geworden, zur Herstellung von Chloraromaten Eisen(III)chlorid zu verwenden (J. Chemical Society 1944; 18-19; J. Chemical Society 1944; 393-5). Das Eisen(III)-chlorid wird dabei jedoch in überstöchiometrischen Mengen verwendet. Auch die Verwendung von Eisen(II)chlorid ist bekannt geworden (Tetrahedron Letters 51 (1970) 4455-8). Ebenfalls ist bereits bekannt geworden, Chloraromaten durch eine Sandmeyer-Reaktion in Gegenwart eines Eisen(II)-salzes in unterstöchiometrischer Mengen herzustellen, wobei jedoch gleichzeitig auch Kupfer(I)chlorid eingesetzt wird (FR-A-2475535). Somit wird das Problem der kupferhaltigen Abfälle auf diese Weise nicht vollständig gelöst.

Die bisher bekannt gewordenen Methoden zur Durchführung der Sandmeyer-Reaktion zur Herstellung von Chlor- oder Bromaromaten weisen demnach z.T. erhebliche Mängel und Nachteile auf, vor allem in Hinblick auf eine Durchführung im technischen Maßstab.

Es wurde nun gefunden, dass die Sandmeyer-Reaktion zur Herstellung von Chlor- und Bromaromaten vorteilhaft so durchgeführt werden kann, dass man in einem ersten Schritt das entsprechenden Anilin der Formel (I) in bekannter Weise in Gegenwart wässriger Salz- oder Bromwasserstoffsäure mittels Natrium- oder Kaliumnitrit diazotiert und anschließend in einem zweiten Schritt durch Zusatz einer Eisen(II)- oder Eisen(III)-Verbindung und gegebenenfalls unter Zusatz von chlorid- oder bromidhaltigen Verbindungen in den Chlor- oder Bromaromaten der Formel (II) überführt. Die Eisen(II)- oder Eisen(III)-Verbindung wird dabei bevorzugt in unterstöchiometrischen Mengen eingesetzt. Die Verwendung von Kupfersalzen, wie Kupfer(I)chlorid ist nicht mehr notwendig.

Das erfindungsgemäße Verfahren zur Herstellung von Verbindungen der Formel (II) kann durch folgendes Schema veranschaulicht werden:

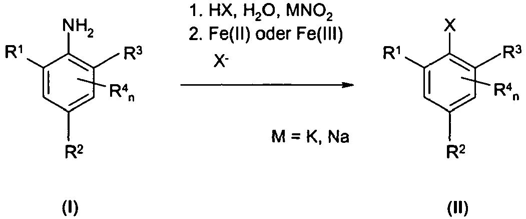

In den Formeln (I) und (II) stehen
X für Chlor oder Brom,
R¹, R² und R³ unabhängig voneinander gleich oder verschieden für Wasserstoff, Halogen, C₁-C₆-Alkyl, C₁-C₆-Halogenalkyl C₂-C₆-Alkenyl, C₂-C₆-Alkinyl, für jeweils gegebenenfalls substituiertes C₃-C₆-Cycloalkyl oder C₄-C₆-Cycloalkenyl, C₁-C₆-Alkoxy, C₁-C₆-Halogenalkoxy, für jeweils gegebenenfalls substituiertes Phenyl, Phenoxy, Heterocyclyl oder Hetaryl, für Cyano, C₁-C₆-Alkylamino oder Di(C₁-C₆-Alkyl)amino,
R⁴ für Halogen, C₁-C₆-Alkyl, C₁-C₆-Halogenalkyl C₂-C₆-Alkenyl, C₂-C₆-Alkinyl, für jeweils gegebenenfalls substituiertes C₃-C₆-Cycloalkyl oder C₄-C₆-Cycloalkenyl, C₁-C₆-Alkoxy, C₁-C₆-Halogenalkoxy, für jeweils gegebenenfalls substituiertes Phenyl, Phenoxy, Heterocyclyl oder Hetaryl, für Cyano, C₁-C₆-Alkylamino oder Di(C₁-C₆-Alkyl)amino,
R¹, R² und R³ unabhängig voneinander gleich oder verschieden bevorzugt für Wasserstoff, Fluor, Chlor, Brom, C₁-C₄-Alkyl, C₂-C₄-Alkenyl, C₂-C₄-Alkinyl, für jeweils gegebenenfalls substituiertes Cyclopropyl oder Cyclopentyl, C₁-C₄-Alkoxy, für jeweils gegebenenfalls substituiertes Phenyl oder Phenoxy oder Cyano,
R⁴ bevorzugt für Fluor, Chlor, Brom, C₁-C₄-Alkyl, C₂-C₄-Alkenyl, C₂-C₄-Alkinyl, für jeweils gegebenenfalls substituiertes Cyclopropyl oder Cyclopentyl, C₁-C₄-Alkoxy, für jeweils gegebenenfalls substituiertes Phenyl oder Phenoxy oder Cyano,
R¹, R² und R³ unabhängig voneinander gleich oder verschieden besonders bevorzugt für Wasserstoff, Chlor, Brom, Methyl, Ethyl, i-Propyl, n-Propyl oder Cyclopropyl,
R⁴ besonders bevorzugt für Chlor, Brom, Methyl, Ethyl, i-Propyl, n-Propyl oder Cyclopropyl,
R¹ ganz besonders bevorzugt für C₁-C₄-Alkyl (hervorgehoben für Methyl, Ethyl oder Isopropyl),
R² ganz besonders bevorzugt für Wasserstoff, C₁-C₄-Alkyl oder Halogen (hervorgehoben für Wasserstoff, Methyl oder Chlor),
R³ ganz besonders bevorzugt für Wasserstoff oder C₁-C₄-Alkyl (hervorgehoben für Wasserstoff, Methyl oder Ethyl),
n für 0, 1 oder 2,
n bevorzugt für 0 oder 1,
n besonders bevorzugt für 0 oder 1,
n ganz besonders bevorzugt für 0.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Iod
**Alkyl:** gesättigte, geradkettige oder verzweigte Kohlenwasserstoffreste mit 1 bis 8 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; Heptyl, Octyl.
**Halogenalkyl:** geradkettige oder verzweigte Alkylgruppen mit 1 bis 8 Kohlenstoffatomen, wobei in diesen Gruppen teilweise oder vollständig die Wasserstoffatome durch Halogenatome wie vorstehend genannt ersetzt sein können, z.B. C₁-C₃-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2-difluorethyl, 2,2-Dichlor-2-fluorethyl, 2,2,2-Trichlorethyl, Pentafluorethyl und 1,1,1-Trifluorprop-2-yl.
**Alkenyl:** ungesättigte, geradkettige oder verzweigte Kohlenwasserstoffreste mit 2 bis 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1,-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.
**Alkinyl:** geradkettige oder verzweigte Kohlenwasserstoffgruppen mit 2 bis 8 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.
**Cycloalkyl:** monocyclische, gesättigte Kohlenwasserstoffgruppen mit 3 bis 8 Kohlenstoffringgliedern, wie Cyclopropyl, Cyclobutyl, Cyclopentyl, Cyclohexyl, Cycloheptyl und Cyclooctyl.
**Cycloalkenyl:** monocyclische, nicht aromatische Kohlenwasserstoffgruppen mit 4 bis 8 Kohlenstoffringgliedern mit mindestens einer Doppelbindung, wie Cyclopenten-1-yl, Cyclohexen-1-yl, Cyclohepta-1,3-dien-1-yl.
**Heterocyclyl/Hetaryl:** unsubstituierter oder substituierter, ungesättigter oder ganz oder teilweise gesättigter heterocyclischer 5- bis 7-gliedrigen Ring, oder ungesättigter oder ganz oder teilweise gesättigter heterocyclischer 3- bis 8-gliedriger Ring, enthaltend bis zu 4 Stickstoffatome oder alternativ 1 Stickstoffatom und bis zu 2 weitere Heteroatome, ausgewählt aus N, O und S: z.B. Oxiranyl, Aziridinyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,3-Dihydrothien-2-yl, 2,3-Dihydrothien-3-yl, 2,4-Dihydrothien-2-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl, 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, 1-Pyrrolyl, 1-Pyrazolyl, 1,2,4-Triazol-1-yl, 1-Imidazolyl, 1,2,3-Triazol-1-yl, 1,3,4-Triazol-1-yl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Überraschenderweise können nach dem erfindungsgemäßen Verfahren die Verbindungen der Formel (II) in besserer Selektivität und in höherer Ausbeute als nach den früher bekannt gewordenen Verfahren hergestellt werden.

Als Lösungsmittel für die Umsetzung nach dem erfindungsgemäßen Verfahren dienen Wasser und wässrige Lösungen von Chlor- oder Bromwasserstoff.

Die Menge an nach dem erfindungsgemäßen Verfahren zur Diazotierung einzusetzendem Chlor- oder Bromwasserstoff kann in weiten Grenzen variiert werden. Man wird mindestens die Menge einsetzen, die entsprechend bekannten Verfahren notwendig ist, um das Anilin der Formel (I) in Gegenwart von Natrium- oder Kaliumnitrit vollständig zu diazotieren.

Die Menge an Natrium- oder Kaliumnitrit zur Diazotierung im ersten Schritt des Verfahrens wird nach bekannten Methoden so gewählt werden, dass nicht mehr als die gerade notwendige Menge eingesetzt wird, oder ein geringer Überschuss, der nach erfolgter Diazotierung in bekannter Weise durch Zusatz von beispielsweise Sulfaminsäure wieder entfernt wird.

Die Diazotierung kann in bekannter Weise bei Temperaturen zwischen -20 und +60°C durchgeführt werden. Bevorzugt sind Temperaturen zwischen -10 und +30°C.

Die Reaktionszeiten des ersten Schritts des Verfahrens betragen zwischen 1 und 6 Stunden.

Im zweiten Schritt des erfindungsgemäßen Verfahrens wird das Diazoniumsalz in Gegenwart eines Eisen(II)- oder Eisen(III)-salzes in den Chlor- oder Bromaromaten der Formel (II) überführt.

Die Menge an nach dem erfindungsgemäßen Verfahren einzusetzender Eisen(II)- oder Eisen(III)-Verbindung ist nicht kritisch. So können beispielsweise 0,005 bis 2 Mol Eisen(II)- oder Eisen(III)-Verbindung pro Mol Anilin eingesetzt werden. Bevorzugt sind 0,01 bis 1 Mol pro Mol Anilin. Besonders bevorzugt sind 0,05 bis 0,75 Mol pro Mol Anilin.

Als Eisen(II)- oder Eisen(III)-Verbindungen seien beispielhaft genannt: Eisen(II)sulfat, Eisen(III)-sulfat, Eisen(II)chlorid, Eisen(III)-chlorid, Eisen(II)-bromid, Eisen(III)-bromid, Eisen(II)-fluorid, Eisen(III)-fluorid, Eisen(II)acetat, Eisen(II)-propionat, Eisen(II)-stearat, Eisen(II)-sulfamat, Eisen(II)-oxalat, Eisen(III)-oxalat, Eisen(III)-citrat, Eisen(II)-gluconat, Eisen(II)-acetylacetonat, Eisen(III)-acetylacetonat, Eisen(III)-nitrat, Eisen(III)-phosphat, Ammoniumeisen(II)-sulfat, Ammoniumeisen(III)-sulfat, Eisen(II,III)-oxid und Eisen(III)-oxid. Bevorzugt sind Eisen(II)sulfat, Eisen(III)-sulfat, Eisen(II)-chlorid, Eisen(III)-chlorid, Eisen(II)-bromid und Eisen(III)-bromid.

Bei der Aufführung dieser Eisenverbindungen sind jeweils auch die existierenden Hydratformen mit gemeint.

Es kann vorteilhaft sein, über den bei der Diazotierung eingesetzten Chlor- oder Bromwasserstoff hinaus im zweiten Schritt des erfindungsgemäßen Verfahrens zusätzliche Mengen Chlor- oder Bromwasserstoff einzusetzen, um eine möglichst weitgehende Umsetzung des Diazoniumsalzes zur Chlor- oder Bromverbindung zu erzielen.

Anstelle von Chlor- oder Bromwasserstoff können an dieser Stelle auch Alkali- oder Erdalkalichloride oder -bromide eingesetzt werden. Als Alkali- oder Erdalkalichloride oder -bromide seien beispielhaft genannt Lithiumchlorid, Natriumchlorid, Kaliumchlorid, Magnesiumchlorid, Calciumchlorid, Lithiumbromid, Natriumbromid,Kaliumbromid und Magnesiumbromid.

Bevorzugt sind Lithiumchlorid, Natriumchlorid, Kaliumchlorid, Lithiumbromid, Natriumbromid und Kaliumbromid.

Die Mengen an im zweiten Schritt des erfindungsgemäßen Verfahrens zusätzlich eingesetzten Chlorids oder Bromids können in weiten Grenzen variiert werden. Üblicherweise liegen sie zwischen 0 und 20 Mol je Mol Anilin der Formel (I), bevorzugt zwischen 0,5 und 15 Mol je Mol Anilin der Formel (I).

Als Lösungsmittel für den zweiten Schritt des erfindungsgemäßen Verfahrens wird bevorzugt Wasser verwendet. Die Menge an Wasser richtet sich nach der Menge und Löslichkeit der jeweils verwendeten Chloride oder Bromide, bzw. resultiert aus der Konzentration der verwendeten wässrigen Chlor- oder Bromwasserstofflösungen. In der Regel wird man zur Erzielung einer hohen Raum-Zeit-Ausbeute diese Wassermenge so klein wie möglich wählen.

Der zweite Schritt des erfindungsgemäßen Verfahrens wird bei Temperaturen zwischen 20 und 120°C durchgeführt. Bevorzugt sind Temperaturen zwischen 30 und 100°C.

Die Reaktionszeiten des zweiten Schritts des erfindungsgemäßen Verfahrens betragen zwischen 1 und 6 Stunden.

Das erfindungsgemäße Verfahren Verfahrens zur Herstellung von Verbindungen der Formel (II) wird bevorzugt so gestaltet, dass die Schritte nacheinander ohne Isolierung des Zwischenproduktes durchgeführt werden.

Die Herstellung von Verbindungen der Formel (II) nach dem erfindungsgemäßen Verfahren soll durch die folgenden Herstellungsbeispiele veranschaulicht werden:

### Beispiel 1:

### 4-Chlor-2,6-dimethyl-brombenzol

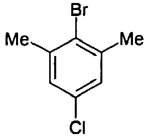

Man legt 65 ml 48%ige wässrige HBr vor und gibt portionsweise 15,56 g [0,1 mol] 4-Chlor-2,6-dimethyl-anilin zu. Die resultierende dicke Suspension wird 15 Minuten bei 80°C gerührt. Danach kühlt man auf -10°C ab und tropft innerhalb von ca. 40 Minuten eine Lösung von 8 g [0,116 mol] NaNO₂ in 35 ml Wasser so zu, dass die Temperatur -5°C nicht überschreitet. Es werden 80 mg Sulfaminsäure zugesetzt. Dann wird die auf -10°C gekühlte Suspension des Diazoniumsalzes innerhalb von etwa 25 Minuten in eine auf 80°C erwärmte Lösung von 28,6 g [0,103 mol] FeSO₄ x 7 H₂O in 65 ml 62%iger wässriger HBr dosiert. Man rührt danach noch 1 Stunde bei 80°C, lässt auf Raumtemperatur abkühlen, versetzt das Reaktionsgemisch mit 125 ml Wasser, trennt die Phasen und extrahiert die wässrige Phase dreimal mit je 50 ml Methylenchlorid. Die vereinigten organischen Phasen werden zweimal mit je 25 ml Wasser gewaschen, getrocknet und unter vermindertem Druck eingeengt. Man erhält 17,2 g eines Öles, das lt. GC 95,6% 4-Chlor-2,6-dimethyl-brombenzol enthält (75% der Theorie).

### Beispiel 2:

### 4-Chlor-2,6-dimethyl-brombenzol

Man geht vor wie in Beispiel 1, setzt jedoch nur 13,9 g [0,05 mol] FeSO₄ x 7 H₂O ein. Man erhält 19,7 g eines Öles, das lt. GC 97,7% 4-Chlor-2,6-dimethyl-brombenzol enthält (87% der Theorie).

### Beispiel 3:

### 4-Chlor-2,6-dimethyl-brombenzol

Man geht vor wie in Beispiel 1, setzt jedoch nur 6,95 g [0,025 mol] FeSO₄ x 7 H₂O ein. Man erhält 20,7 g eines Öles, das lt. GC 97,1% 4-Chlor-2,6-dimethyl-brombenzol enthält (91% der Theorie).

### Beispiel 4:

### 4-Chlor-2,6-dimethyl-brombenzol

Man geht vor wie in Beispiel 1, setzt jedoch nur 3,475 g [0,0125 mol] FeSO₄ x 7 H₂O ein. Man erhält 21,5 g eines Öles, das lt. GC 92% 4-Chlor-2,6-dimethyl-brombenzol enthält (90% der Theorie).

### Beispiel 5:

### 4-Chlor-2,6-dimethyl-brombenzol

Man geht vor wie in Beispiel 1, setzt jedoch nur 1,668 g [0,006mol] FeSO₄ x 7 H₂O ein. Man erhält 21,4 g eines Öles, das lt. GC 84,8% 4-Chlor-2,6-dimethyl-brombenzol enthält (83% der Theorie).

### Vergleichsbeispiel 1:

### 4-Chlor-2,6-dimethyl-brombenzol

Man legt 75 ml 48%ige wässrige HBr vor und gibt portionsweise 19,67 g [0,12 mol] 4-Chlor-2,6-dimethyl-anilin zu. Die resultierende dicke Suspension wird 15 Minuten bei 80°C gerührt. Danach kühlt man auf -10°C ab und tropft innerhalb von ca. 1 h eine Lösung von 9,6 g [0,139 mol] NaNO₂ in 45 ml Wasser so zu, dass die Temperatur -5°C nicht überschreitet. Es werden 100 mg Sulfaminsäure zugesetzt. Dann wird die auf -10°C gekühlte Suspension des Diazoniumsalzes innerhalb von etwa 35 Minuten in eine auf 80°C erwärmte Lösung von 17,93 g [0,125 mol] Cu(I)Br in 75 ml 62%iger wässriger HBr dosiert. Man rührt danach noch 1 Stunde bei 80°C, lässt auf Raumtemperatur abkühlen, versetzt das Reaktionsgemisch mit 125 ml Wasser, trennt die Phasen und extrahiert die wässrige Phase dreimal mit je 50 ml Methylenchlorid. Die vereinigten organischen Phasen werden zweimal mit je 25 ml Wasser gewaschen, getrocknet und unter vermindertem Druck eingeengt. Man erhält 20,2 g eines Öles, das lt. GC 95,5% 4-Chlor-2,6-dimethyl-brombenzol enthält (73% der Theorie).

### Vergleichsbeispiel 2 :

### 4-Chlor-2,6-dimethyl-brombenzol

Man geht vor wie in Vergleichsbeispiel 1, setzt jedoch nur 8,607 g [0,06 mol] Cu(I)Br ein. Man erhält 23,6 g eines Öles, das lt. GC 81,9% 4-Chlor-2,6-dimethyl-brombenzol enthält (73% der Theorie).

### Vergleichbeispiel 3:

### 4-Chlor-2,6-dimethyl-brombenzol

Man geht vor wie in Vergleichsbeispiel 1, setzt jedoch nur 4,304 g [0,03 mol] Cu(I)Br ein. Man erhält 23,7 g eines Öles, das lt. GC 82% 4-Chlor-2,6-dimethyl-brombenzol enthält (74% der Theorie).

### Beispiel 6:

### 2,6-Diethyl-4-methyl-brombenzol

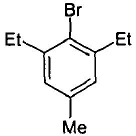

Man legt 65 ml 48%ige wässrige HBr vor und gibt portionsweise 16,33 g [0,1 mol] 2,6-Diethyl-4-methyl-anilin zu. Die resultierende dicke Suspension wird 15 Minuten bei 80°C gerührt. Danach kühlt man auf -10°C ab und tropft innerhalb von ca. 1 h eine Lösung von 8 g [0,116 mol] NaNO₂ in 35 ml Wasser so zu, dass die Temperatur -5°C nicht überschreitet. Es werden 80 mg Sulfaminsäure zugesetzt. Dann wird die auf -10°C gekühlte Suspension des Diazoniumsalzes innerhalb von etwa 30 Minuten in eine auf 80°C erwärmte Lösung von 13,9 g [0,05 mol] FeSO₄ x 7 H₂O in 65 ml 62%iger wässriger HBr dosiert. Man rührt danach noch 1 Stunde bei 80°C, lässt auf Raumtemperatur abkühlen, versetzt das Reaktionsgemisch mit 125 ml Wasser, trennt die Phasen und extrahiert die wässrige Phase dreimal mit je 50 ml Methylenchlorid. Die vereinigten organischen Phasen werden zweimal mit je 25 ml Wasser gewaschen, getrocknet und unter vermindertem Druck eingeengt. Man erhält 21,3 g eines Öles, das lt. GC 94,6% 2,6-Diethyl-4-methyl-brombenzol enthält (89% der Theorie).

### Beispiel 7:

### 2,6-Diethyl-4-methyl-brombenzol

Man geht vor wie Beispiel 6, verwendet im zweiten Schritt des Verfahrens jedoch anstatt der Bromwasserstoffsäure eine Lösung von 7,5 g [0,75 mol] NaBr in 70 ml Wasser. Man erhält 21,9 g eines Öles, das lt. GC 93,3% 2,6-Diethyl-4-methyl-brombenzol enthält (90% der Theorie).

### Beispiel 8:

### 2,6-Dimethyl-brombenzol

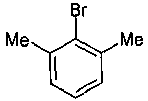

Man legt 65 ml 48%ige wässrige HBr vor und gibt portionsweise 12,12 g [0,1 mol] 2,6-Dimethylanilin zu. Die resultierende dicke Suspension wird 15 Minuten bei 80°C gerührt. Danach kühlt man auf -10°C ab und tropft innerhalb von ca. 1 h eine Lösung von 8 g [0,116 mol] NaNO₂ in 35 ml Wasser so zu, dass die Temperatur -5°C nicht überschreitet. Es werden 80 mg Sulfaminsäure zugesetzt. Dann wird die auf -10°C gekühlte Suspension des Diazoniumsalzes innerhalb von etwa 30 Minuten in eine auf 80°C erwärmte Lösung von 13,9 g [0,05 mol] FeSO₄ x 7 H₂O in 65 ml 62%iger wässriger HBr dosiert. Man rührt danach noch 1 Stunde bei 80°C, lässt auf Raumtemperatur abkühlen, versetzt das Reaktionsgemisch mit 125 ml Wasser, trennt die Phasen und extrahiert die wässrige Phase dreimal mit je 50 ml Methylenchlorid. Die vereinigten organischen Phasen werden zweimal mit je 25 ml Wasser gewaschen, getrocknet und unter vermindertem Druck eingeengt. Man erhält 17,4 g eines Öles, das lt. GC 83% 2,6-Dimethyl-brombenzol enthält (78% der Theorie).

### Vergleichsbeispiel 4:

### 2,6-Dimethyl-brombenzol

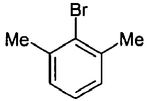

Man legt 125 ml 48%ige wässrige HBr vor und gibt portionsweise 24,24 g [0,2 mol] 2,6-Dimethylanilin zu. Die resultierende dicke Suspension wird 15 Minuten bei 80°C gerührt. Danach kühlt man auf -10°C ab und tropft innerhalb von ca. 1 h eine Lösung von 16 g [0,232 mol] NaNO₂ in 70 ml Wasser so zu, dass die Temperatur -5°C nicht überschreitet. Es werden 160 mg Sulfaminsäure zugesetzt. Dann wird die auf -10°C gekühlte Suspension des Diazoniumsalzes innerhalb von etwa 45 Minuten in eine auf 60°C erwärmte Lösung von 31,6 g [0,22 mol] Cu(I)Br in 130 ml 62%iger wässriger HBr dosiert. Man rührt danach noch 1 Stunde bei 80°C, lässt auf Raumtemperatur abkühlen, versetzt das Reaktionsgemisch mit 250 ml Wasser, trennt die Phasen und extrahiert die wässrige Phase dreimal mit je 100 ml Methylenchlorid. Die vereinigten organischen Phasen werden mit je 50 ml Wasser und gesättigter wässriger NaCl-Lösung gewaschen, getrocknet und unter vermindertem Druck eingeengt. Man erhält 33,3 g eines Öles, das lt. GC 75,6% 2,6-Dimethyl-brombenzol enthält (68% der Theorie).

### Beispiel 9:

### 2,6-Diethyl-4-methyl-chlorbenzol

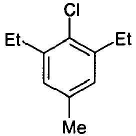

Man legt 65 ml 36%ige wässrige HCl vor und gibt portionsweise 16,33 g [0,1 mol] 2,6-Diethyl-4-methyl-anilin zu. Die resultierende dicke Suspension wird 5 Minuten bei 65°C gerührt. Danach kühlt man auf -10°C ab und tropft innerhalb von ca. 1 h eine Lösung von 8 g [0,116 mol] NaNO₂ in 35 ml Wasser so zu, dass die Temperatur -5°C nicht überschreitet. Es werden 80 mg Sulfaminsäure zugesetzt. Dann wird die auf -10°C gekühlte Suspension des Diazoniumsalzes innerhalb von etwa 30 Minuten in eine auf 70°C erwärmte Lösung von 13,9 g [0,05 mol] FeSO₄ x 7 H₂O in 65 ml 36%iger wässriger HCl dosiert. Man rührt danach noch 1 Stunde bei 65-75°C, lässt auf Raumtemperatur abkühlen, versetzt das Reaktionsgemisch mit 200 ml Wasser, trennt die Phasen und extrahiert die wässrige Phase dreimal mit je 50 ml Methylenchlorid. Die vereinigten organischen Phasen werden zweimal mit je 25 ml Wasser gewaschen, getrocknet und unter vermindertem Druck eingeengt. Man erhält 17,3 g eines Öles, das lt. GC 87,6% 2,6-Diethyl-4-methyl-chlorbenzol enthält (83% der Theorie).

### Beispiel 10:

### 2,6-Diethyl-4-methyl-chlorbenzol

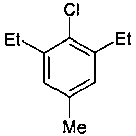

Man legt 65 ml 36%ige wässrige HCl vor und gibt portionsweise 16,33 g [0,1 mol] 2,6-Diethyl-4-methyl-anilin zu. Die resultierende dicke Suspension wird 5 Minuten bei 65°C gerührt. Danach kühlt man auf -10°C ab und tropft innerhalb von ca. 1 h eine Lösung von 8 g [0,116 mol] NaNO₂ in 35 ml Wasser so zu, dass die Temperatur -5°C nicht überschreitet. Es werden 80 mg Sulfaminsäure zugesetzt. Dann wird die auf -10°C gekühlte Suspension des Diazoniumsalzes innerhalb von etwa 30 Minuten in eine auf 70°C erwärmte Lösung von 8,36 g [0,05 mol] FeCl₃ in 65 ml 36%iger wässriger HCl dosiert. Man rührt danach noch 1 Stunde bei 65-75°C, lässt auf Raumtemperatur abkühlen, versetzt das Reaktionsgemisch mit 200 ml Wasser, trennt die Phasen und extrahiert die wässrige Phase dreimal mit je 50 ml Methylenchlorid. Die vereinigten organischen Phasen werden zweimal mit je 25 ml Wasser gewaschen, getrocknet und unter vermindertem Druck eingeengt. Man erhält 18,1 g eines Öles, das lt. GC 89% 2,6-Diethyl-4-methyl-chlorbenzol enthält (88% der Theorie).

### Beispiel 11:

### 2,6-Diethyl-4-methyl-chlorbenzol

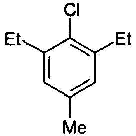

Man legt 65 ml 36%ige wässrige HCl vor und gibt portionsweise 16,33 g [0,1 mol] 2,6-Diethyl-4-methyl-anilin zu. Die resultierende dicke Suspension wird 5 Minuten bei 50°C gerührt. Danach kühlt man auf -10°C ab und tropft innerhalb von ca. 1 h eine Lösung von 8 g [0,116 mol] NaNO₂ in 35 ml Wasser so zu, dass die Temperatur -5°C nicht überschreitet. Es werden 80 mg Sulfaminsäure zugesetzt. Dann wird die auf -10°C gekühlte Suspension des Diazoniumsalzes innerhalb von etwa 30 Minuten in eine auf 65°C erwärmte Lösung von 4,18 g [0,025 mol] FeCl₃ in 65 ml 36%iger wässriger HCl dosiert. Man rührt danach noch 1 Stunde bei 65°C, lässt auf Raumtemperatur abkühlen, versetzt das Reaktionsgemisch mit 200 ml Wasser, trennt die Phasen und extrahiert die wässrige Phase dreimal mit je 50 ml Methylenchlorid. Die vereinigten organischen Phasen werden zweimal mit je 25 ml Wasser gewaschen, getrocknet und unter vermindertem Druck eingeengt. Man erhält 17,9 g eines Öles, das lt. GC 93,1% 2,6-Diethyl-4-methyl-chlorbenzol enthält (91% der Theorie).

### Beispiel 12:

### 2,6-Diethyl-4-methyl-chlorbenzol

Man verfährt wie in Beispiel 10, setzt im zweiten Schritt jedoch anstatt der Salzsäure eine Lösung von 7,85 g [0,785 mol] LiCl in 40 ml Wasser ein. Man erhält 17 g eines Öles, das lt. GC 90,3% 2,6-Diethyl-4-methyl-chlorbenzol enthält (84% der Theorie).

### Beispiel 13:

### 2-Isopropyl-brombenzol

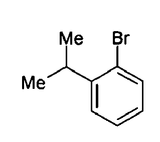

Man legt 65 ml 48%ige wässrige HBr vor und gibt portionsweise 13,5 g [0,1 mol] 2-Isopropyl-anilin zu. Die resultierende dicke Suspension wird 15 Minuten bei 80°C gerührt. Danach kühlt man auf -10°C ab und tropft innerhalb von ca. 1 h eine Lösung von 8 g [0,116 mol] NaNO₂ in 35 ml Wasser so zu, dass die Temperatur -5°C nicht überschreitet. Es werden 80 mg Sulfaminsäure zugesetzt. Dann wird die auf -10°C gekühlte dünne Suspension des Diazoniumsalzes innerhalb von etwa 30 Minuten in eine auf 70°C erwärmte Lösung von 13,9 g [0,05 mol] FeSO₄ x 7 H₂O in 65 ml 62%iger wässriger HBr dosiert. Man rührt danach noch 1 Stunde ohne weiteres Erwärmen und lässt auf Raumtemperatur abkühlen, versetzt das Reaktionsgemisch mit 125 ml Wasser, trennt die Phasen und extrahiert die wässrige Phase dreimal mit je 50 ml Methylenchlorid. Die vereinigten organischen Phasen werden zweimal mit je 25 ml Wasser gewaschen, getrocknet und unter vermindertem Druck eingeengt. Man erhält 18,75 g eines Öles, das lt. GC 82,3% 2-Isopropyl-brombenzol enthält (77,5% der Theorie).

### Vergleichsbeispiel 5:

### 2-Isopropyl-brombenzol

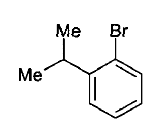

Man verfährt wie in Beispiel 13, führt dabei die Reaktion jedoch in Gegenwart von 7,17 g [0,05 mol] Cu(I)Br anstelle von FeSO₄ x 7 H₂O durch. Es resultieren 18,2 g eines Öles, das lt. GC 65,4% 2-Isopropyl-brombenzol enthält (65,4% der Theorie).

## Patentansprüche

1. Verfahren zur Herstellung von Verbindungen der Formel (II) 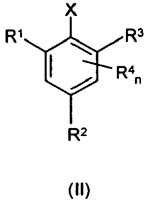 in der
X für Chlor oder Brom,
R¹, R² und R³ unabhängig voneinander gleich oder verschieden für Wasserstoff, Halogen, C₁-C₆-Alkyl, C₁-C₆-Halogenalkyl C₂-C₆-Alkenyl, C₂-C₆-Alkinyl, für jeweils gegebenenfalls substituiertes C₃-C₆-Cycloalkyl oder C₄-C₆-Cycloalkenyl, C₁-C₆-Alkoxy, C₁-C₆-Halogenalkoxy, für jeweils gegebenenfalls substituiertes Phenyl, Phenoxy, Heterocyclyl oder Hetaryl, für Cyano, C₁-C₆-Alkylamino oder Di(C₁-C₆-Alkyl)amino,
R⁴ für Halogen, C₁-C₆-Alkyl, C₁-C₆-Halogenalkyl C₂-C₆-Alkenyl, C₂-C₆-Alkinyl, für jeweils gegebenenfalls substituiertes C₃-C₆-Cycloalkyl oder C₄-C₆-Cycloalkenyl, C₁-C₆-Alkoxy, C₁-C₆-Halogenalkoxy, für jeweils gegebenenfalls substituiertes Phenyl, Phenoxy, Heterocyclyl oder Hetaryl, für Cyano, C₁-C₆-Alkylamino oder Di(C₁-C₆-Alkyl)amino,
n für 0, 1 oder 2
stehen,
**dadurch gekennzeichnet, dass** man die Verbindung der Formel (I) 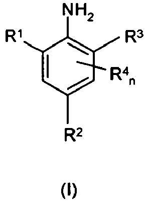 in der R¹, R², R³, R⁴ und n die oben genannten Bedeutungen haben,
in Gegenwart wässriger Chlor- oder Bromwasserstoffsäuren mittels Natrium- oder Kaliumnitrit diazotiert und anschließend durch Zusatz einer Eisen(II)- oder Eisen(III)-Verbindung und gegebenfalls zusätzlicher Mengen an Chlor- oder Bromwasserstoff oder Alkali- oder Erdalkalichloride oder -bromide umsetzt, wobei Kupfesalze nicht zum Einsatz kommen.

2. Verfahren zur Herstellung von Verbindungen der Formel (II) gemäß Anspruch 1, in welcher
X für Chlor oder Brom steht,
R¹, R² und R³ unabhängig voneinander gleich oder verschieden für Wasserstoff, Halogen, C₁-C₆-Alkyl, C₁-C₆-Halogenalkyl C₂-C₆-Alkenyl, C₂-C₆-Alkinyl, für jeweils gegebenenfalls substituiertes C₃-C₆-Cycloalkyl oder C₄-C₆-Cycloalkenyl, C₁-C₆-Alkoxy, C₁-C₆-Halogenalkoxy, für jeweils gegebenenfalls substituiertes Phenyl, Phenoxy, Heterocyclyl oder Hetaryl, für Cyano, C₁-C₆-Alkylamino oder Di(C₁-C₆-Alkyl)amino stehen,
R⁴ für Halogen, C₁-C₆-Alkyl, C₁-C₆-Halogenalkyl C₂-C₆-Alkenyl, C₂-C₆-Alkinyl, für jeweils gegebenenfalls substituiertes C₃-C₆-Cycloalkyl oder C₄-C₆-Cycloalkenyl, C₁-C₆-Alkoxy, C₁-C₆-Halogenalkoxy, für jeweils gegebenenfalls substituiertes Phenyl, Phenoxy, Heterocyclyl oder Hetaryl, für Cyano, C₁-C₆-Alkylamino oder Di(C₁-C₆-Alkyl)amino stehen,
n für 0, 1 oder 2 steht.

3. Verfahren zur Herstellung von Verbindungen der Formel (II) gemäß Anspruch 1, in welcher
X für Chlor oder Brom steht,
R¹, R² und R³ unabhängig voneinander gleich oder verschieden für Wasserstoff, Fluor, Chlor, Brom, C₁-C₄-Alkyl, C₂-C₄-Alkenyl, C₂-C₄-Alkinyl, für jeweils gegebenenfalls substituiertes Cyclopropyl oder Cyclopentyl, C₁-C₄-Alkoxy, für jeweils gegebenenfalls substituiertes Phenyl oder Phenoxy oder Cyano stehen,
R⁴ für Fluor, Chlor, Brom, C₁-C₄-Alkyl, C₂-C₄-Alkenyl, C₂-C₄-Alkinyl, für jeweils gegebenenfalls substituiertes Cyclopropyl oder Cyclopentyl, C₁-C₄-Alkoxy, für jeweils gegebenenfalls substituiertes Phenyl oder Phenoxy oder Cyano steht,
n für 0 oder 1 steht.

4. Verfahren zur Herstellung von Verbindungen der Formel (II) gemäß Anspruch 1, in welcher
X für Chlor oder Brom steht,
R¹, R² und R³ unabhängig voneinander gleich oder verschieden für Wasserstoff, Chlor, Brom, Methyl, Ethyl, i-Propyl, n-Propyl oder Cyclopropyl stehen,
R⁴ für Chlor, Brom, Methyl, Ethyl, i-Propyl, n-Propyl oder Cyclopropyl steht,
n für 0 oder 1 steht.

5. Verfahren zur Herstellung von Verbindungen der Formel (II) gemäß Anspruch 1, in welcher
X für Chlor oder Brom steht,
R¹ für C₁-C₄-Alkyl steht,
R² für Wasserstoff, C₁-C₄-Alkyl oder Halogen steht,
R³ für Wasserstoff oder C₁-C₄-Alkyl steht,
n für 0 steht.

6. Verfahren gemäß Anspruch 1, **dadurch gekennzeichnet, dass** die Eisen(II)- oder Eisen(III)-Verbindung in unterstöchiometrischer Menge eingesetzt wird.

7. Verfahren gemäß Anspruch 6, **dadurch gekennzeichnet, dass** die Eisen(II)- oder Eisen(III)-Verbindung in Mengen von 0,01 bis 1 Mol pro Mol Anilin der Formel (I) eingesetzt wird.

8. Verfahren gemäß Anspruch 6, **dadurch gekennzeichnet, dass** die Eisen(II)- oder Eisen(III)-Verbindung in Mengen von 0.05 bis 0.75 Mol pro Mol Anilin der Formel (I) eingesetzt wird.

9. Verfahren gemäß Anspruch 1, **dadurch gekennzeichnet, dass** als Eisenverbindung Eisen(II)sulfat, Eisen(III)-sulfat, Eisen(II)chlorid, Eisen(III)-chlorid, Eisen(II)-bromid, Eisen(III)-bromid, Eisen(II)-fluorid, Eisen(III)-fluorid, Eisen(11)acetat, Eisen(II)-propionat, Eisen(II)-stearat, Eisen(II)-sulfamat, Eisen(II)-oxalat, Eisen(III)-oxalat, Eisen(III)-citrat, Eisen(II)-gluconat, Eisen(II)-acetylacetonat, Eisen(III)-acetylacetonat, Eisen(III)-nitrat, Eisen(111)-phosphat, Ammoniumeisen(II)-sulfat, Ammoniumeisen(III)-sulfat, Eisen(II,III)-oxid, Eisen(III)-oxid oder, soweit existierend, die jeweiligen Hydrate verwendet werden.

10. Verfahren gemäß Anspruch 9, **dadurch gekennzeichnet, dass** als Eisenverbindung Eisen(II)sulfat, Eisen(III)-sulfat, Eisen(11)-chlorid, Eisen(III)-chlorid, Eisen(II)-bromid, Eisen(III)-bromid oder, soweit existierend, die jeweiligen Hydrate verwendet werden.

11. Verfahren gemäß Anspruch 1, **dadurch gekennzeichnet, dass** im zweiten Schritt als zusätzliche Chlorid- oder Bromidquelle ein Alkali- oder Erdalkalichlorid oder -bromid verwendet wird.

12. Verfahren gemäß Anspruch 11, **dadurch gekennzeichnet, dass** Lithiumchlorid, Natriumchlorid, Kaliumchlorid, Magnesiumchlorid, Calciumchlorid, Lithiumbromid, Natriumbromid,Kaliumbromid oder Magnesiumbromid verwendet werden.

13. Verfahren gemäß Anspruch 1, **dadurch gekennzeichnet, dass** im zweiten Schritt als zusätzliche Chlorid- oder Bromidquelle Chlor- oder Bromwasserstoff verwendet wird.

## Claims

1. Process for preparing compounds of the formula (II) 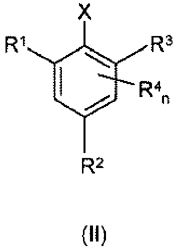 in which
X is chlorine or bromine,
R¹, R² and R³ are the same or different and are independently hydrogen, halogen, C₁-C₆-alkyl, C₁-C₆-haloalkyl, C₂-C₆-alkenyl, C₂-C₆-alkynyl, optionally substituted C₃-C₆-cycloalkyl or C₄-C₆-cycloalkenyl, C₁-C₆-alkoxy, C₁-C₆-haloalkoxy, optionally substituted phenyl, phenoxy, heterocyclyl or hetaryl, or cyano, C₁-C₆-alkylamino or di(C₁-C₆-alkyl)amino,
R4 is halogen, C1-C6-alkyl, C1-C6-haloalkyl, C2-C6-alkenyl, C2-C6-alkynyl, optionally substituted C3-C6-cycloalkyl or C4-C6-cycloalkenyl, C1-C6-alkoxy, C1-C6-haloalkoxy, optionally substituted phenyl, phenoxy, heterocyclyl or hetaryl, or cyano, C1-C6-alkylamino or di(C1-C6-alkyl)amino,
n is 0, 1 or 2,
**characterized in that** the compound of the formula (I) 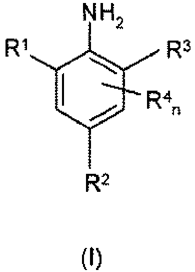 in which R1, R2, R3, R4 and n are each as defined above
is diazotized by means of sodium nitrite or potassium nitrite in the presence of aqueous hydrochloric or hydrobromic acids and then converted by addition of an iron(II) or iron(III) compound and optionally additional amounts of hydrogen chloride or hydrogen bromide or alkali metal or alkaline earth metal chlorides or bromides, wherein copper salts are not used.

2. Process for preparing compounds of the formula (II) according to Claim 1, in which
X is chlorine or bromine,
R1, R2 and R3 are the same or different and are independently hydrogen, halogen, C1-C6-alkyl, C1-C6-haloalkyl, C2-C6-alkenyl, C2-C6-alkynyl, optionally substituted C3-C6-cycloalkyl or C4-C6-cycloalkenyl, C1-C6-alkoxy, C1-C6-haloalkoxy, optionally substituted phenyl, phenoxy, heterocyclyl or hetaryl, or cyano, C1-C6-alkylamino or di(C1-C6-alkyl)amino,
R4 is halogen, C1-C6-alkyl, C1-C6-haloalkyl, C2-C6-alkenyl, C2-C6-alkynyl, optionally substituted C3-C6-cycloalkyl or C4-C6-cycloalkenyl, C1-C6-alkoxy, C1-C6-haloalkoxy, optionally substituted phenyl, phenoxy, heterocyclyl or hetaryl, or cyano, C1-C6-alkylamino or di(C1-C6-alkyl)amino,
n is 0, 1 or 2.

3. Process for preparing compounds of the formula (II) according to Claim 1, in which
X is chlorine or bromine,
R1, R2 and R3 are the same or different and are independently hydrogen, fluorine, chlorine, bromine, C1-C4-alkyl, C2-C4-alkenyl, C2-C4-alkynyl, optionally substituted cyclopropyl or cyclopentyl, C1-C4-alkoxy, optionally substituted phenyl or phenoxy, or cyano,
R4 is fluorine, chlorine, bromine, C1-C4-alkyl, C2-C4-alkenyl, C2-C4-alkynyl, optionally substituted cyclopropyl or cyclopentyl, C1-C4-alkoxy, optionally substituted phenyl or phenoxy, or cyano,
n is 0 or 1.

4. Process for preparing compounds of the formula (II) according to Claim 1, in which
X is chlorine or bromine,
R1, R2 and R3 are the same or different and are independently hydrogen, chlorine, bromine, methyl, ethyl, i-propyl, n-propyl or cyclopropyl,
R4 is chlorine, bromine, methyl, ethyl, i-propyl, n-propyl or cyclopropyl,
n is 0 or 1.

5. Process for preparing compounds of the formula (II) according to Claim 1, in which
X is chlorine or bromine,
R1 is C1-C4-alkyl,
R2 is hydrogen, C1-C4-alkyl or halogen,
R3 is hydrogen or C1-C4-alkyl,
n is 0.

6. Process according to Claim 1, **characterized in that** the iron(II) or iron(III) compound is used in a substoichiometric amount.

7. Process according to Claim 6, **characterized in that** the iron(II) or iron(III) compound is used in amounts of 0.01 to 1 mol per mole of aniline of the formula (I).

8. Process according to Claim 6, **characterized in that** the iron(II) or iron(III) compound is used in amounts of 0.05 to 0.75 mol per mole of aniline of the formula (I).

9. Process according to Claim 1, **characterized in that** the iron compound used is iron (II) sulphate, iron(III) sulphate, iron(II) chloride, iron(III) chloride, iron(II) bromide, iron(III) bromide, iron(II) fluoride, iron(III) fluoride, iron(II) acetate, iron(II) propionate, iron(II) stearate, iron(II) sulphamate, iron(II) oxalate, iron(III) oxalate, iron(III) citrate, iron(II) gluconate, iron(II) acetylacetonate, iron(III) acetylacetonate, iron(III) nitrate, iron(III) phosphate, ammonium iron(II) sulphate, ammonium iron(III) sulphate, iron(II,III) oxide, iron(III) oxide or, where they exist, the particular hydrates.

10. Process according to Claim 9, **characterized in that** the iron compound used is iron (II) sulphate, iron(III) sulphate, iron(II) chloride, iron(III) chloride, iron(II) bromide, iron(III) bromide or, where they exist, the particular hydrates.

11. Process according to Claim 1, **characterized in that** the additional chloride or bromide source used in the second step is an alkali metal or alkaline earth metal chloride or bromide.

12. Process according to Claim 11, **characterized in that** lithium chloride, sodium chloride, potassium chloride, magnesium chloride, calcium chloride, lithium bromide, sodium bromide, potassium bromide or magnesium bromide is used.

13. Process according to Claim 1, **characterized in that** the additional chloride or bromide source used in the second step is hydrogen chloride or hydrogen bromide.

## Revendications

1. Procédé pour la préparation de composés de formule (II) 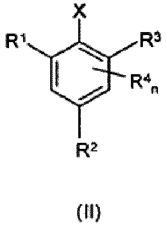 dans laquelle
X représente un atome de chlore ou de brome,
R¹, R² et R³, identiques ou différents, représentent chacun indépendamment un atome d'hydrogène ou d'halogène, un groupe alkyle en C₁-C₆, halogénoalkyle(C₁-C₆), alcényle en C₂-C₆, alcynyle en C₂-C₆, un groupe cycloalkyle en C₃-C₆ ou cycloalcényle en C₄-C₆ chacun éventuellement substitué, un groupe alcoxy en C₁-C₆, halogénoalcoxy(C₁-C₆), un groupe phényle, phénoxy, hétérocyclyle ou hétéroaryle chacun éventuellement substitué, un groupe cyano, alkyl(C₁-C₆)amino ou di(alkyl(C₁-C₆))amino,
R⁴ représente un atome d'halogène, un groupe alkyle en C₁-C₆, halogénoalkyle(C₁-C₆), alcényle en C₂-C₆, alcynyle en C₂-C₆, un groupe cycloalkyle en C₃-C₆ ou cycloalcényle en C₄-C₆ chacun éventuellement substitué, un groupe alcoxy en C₁-C₆, halogénoalcoxy(C₁-C₆), un groupe phényle, phénoxy, hétérocyclyle ou hétéroaryle chacun éventuellement substitué, un groupe cyano, alkyl(C₁-C₆)amino ou di(alkyl(C₁-C₆))amino,
n représente 0, 1 ou 2,
**caractérisé en ce qu'**on soumet à une diazotation au moyen de nitrite de sodium ou de potassium le composé de formule (I) 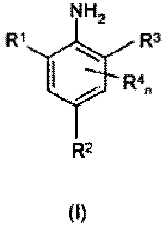 dans laquelle R¹, R², R³, R⁴ et n ont les significations indiquées plus haut,
en présence de solutions aqueuses d'acide chlorhydrique ou bromhydrique et ensuite on le convertit par addition d'un composé contenant du fer(II) ou du fer(III) et éventuellement de quantités supplémentaires d'acide chlorhydrique ou bromhydrique ou de chlorures ou bromures de métaux alcalins ou alcalino-terreux, sans utilisation de sels de cuivre.

2. Procédé pour la préparation de composés de formule (II) selon la revendication 1, dans lesquels
X représente un atome de chlore ou de brome,
R¹, R² et R³, identiques ou différents, représentent chacun indépendamment un atome d'hydrogène ou d'halogène, un groupe alkyle en C₁-C₆, halogénoalkyle(C₁-C₆), alcényle en C₂-C₆, alcynyle en C₂-C₆, un groupe cycloalkyle en C₃-C₆ ou cycloalcényle en C₄-C₆ chacun éventuellement substitué, un groupe alcoxy en C₁-C₆, halogénoalcoxy(C₁-C₆), un groupe phényle, phénoxy, hétérocyclyle ou hétéroaryle chacun éventuellement substitué, un groupe cyano, alkyl(C₁-C₆)amino ou di(alkyl(C₁-C₆))amino,
R⁴ représente un atome d'halogène, un groupe alkyle en C₁-C₆, halogénoalkyle(C₁-C₆), alcényle en C₂-C₆, alcynyle en C₂-C₆, un groupe cycloalkyle en C₃-C₆ ou cycloalcényle en C₄-C₆ chacun éventuellement substitué, un groupe alcoxy en C₁-C₆, halogénoalcoxy(C₁-C₆), un groupe phényle, phénoxy, hétérocyclyle ou hétéroaryle chacun éventuellement substitué, un groupe cyano, alkyl(C₁-C₆)amino ou di(alkyl(C₁-C₆))amino,
n représente 0, 1 ou 2.

3. Procédé pour la préparation de composés de formule (II) selon la revendication 1, dans lesquels
X représente un atome de chlore ou de brome,
R¹, R² et R³, identiques ou différents, représentent chacun indépendamment un atome d'hydrogène, de fluor, chlore, brome, un groupe alkyle en C₁-C₄, alcényle en C₂-C₄, alcynyle en C₂-C₄, un groupe cyclopropyle ou cyclopentyle chacun éventuellement substitué, un groupe alcoxy en C₁-C₄, un groupe phényle ou phénoxy chacun éventuellement substitué ou un groupe cyano,
R⁴ représente un atome de fluor, chlore, brome, un groupe alkyle en C₁-C₄, alcényle en C₂-C₄, alcynyle en C₂-C₄, un groupe cyclopropyle ou cyclopentyle chacun éventuellement substitué, un groupe alcoxy en C₁-C₄, un groupe phényle ou phénoxy chacun éventuellement substitué ou un groupe cyano,
n représente 0 ou 1.

4. Procédé pour la préparation de composés de formule (II) selon la revendication 1, dans lesquels
X représente un atome de chlore ou de brome,
R¹, R² et R³, identiques ou différents, représentent chacun indépendamment un atome d'hydrogène, de chlore, de brome, un groupe méthyle, éthyle, isopropyle, n-propyle ou cyclopropyle,
R⁴ représente un atome de chlore, de brome, un groupe méthyle, éthyle, isopropyle, n-propyle ou cyclopropyle,
n représente 0 ou 1.

5. Procédé pour la préparation de composés de formule (II) selon la revendication 1, dans lesquels
X représente un atome de chlore ou de brome,
R¹ représente un groupe alkyle en C₁-C₄,
R² représente un atome d'hydrogène, un groupe alkyle en C₁-C₄ ou un atome d'halogène,
R³ représente un atome d'hydrogène ou un groupe alkyle en C₁-C₄,
n représente 0.

6. Procédé selon la revendication 1, **caractérisé en ce que** le composé contenant du fer(II) ou du fer(III) est utilisé en quantité sous-stoechiométrique.

7. Procédé selon la revendication 6, **caractérisé en ce que** le composé contenant du fer(II) ou du fer (III) est utilisé en quantités de 0,01 à 1 mole par mole d'aniline de formule (I).

8. Procédé selon la revendication 6, **caractérisé en ce que** le composé contenant du fer(II) ou du fer (III) est utilisé en quantités de 0,05 à 0,75 mole par mole d'aniline de formule (I).

9. Procédé selon la revendication 1, **caractérisé en ce qu'**on utilise comme composé contenant du fer le sulfate de fer(II), le sulfate de fer(III), le chlorure de fer(II), le chlorure de fer(III), le bromure de fer(II), le bromure de fer(III), le fluorure de fer(II), le fluorure de fer(III), l'acétate de fer(II), le propionate de fer(II), le stéarate de fer(II), le sulfamate de fer(II), l'oxalate de fer(II), l'oxalate de fer(III), le citrate de fer(III), le gluconate de fer(II), l'acétylacétonate de fer(II), l'acétyl-acétonate de fer(III), le nitrate de fer(III), le phosphate de fer(III), le sulfate de fer(II) et d'ammonium, le sulfate de fer(III) et d'ammonium, l'oxyde de fer(II,III), l'oxyde de fer(III), ou, s'ils existent, les hydrates respectifs.

10. Procédé selon la revendication 9, **caractérisé en ce qu'**on utilise comme composé contenant du fer le sulfate de fer(II), le sulfate de fer(III), le chlorure de fer(II), le chlorure de fer(III), le bromure de fer(II), le bromure de fer(III) ou, s'ils existent, les hydrates respectifs.

11. Procédé selon la revendication 1, **caractérisé en ce que** dans la deuxième étape on utilise comme source de chlorure ou bromure supplémentaire un chlorure ou bromure de métal alcalin ou alcalino-terreux.

12. Procédé selon la revendication 11, **caractérisé en ce qu'**on utilise le chlorure de lithium, le chlorure de sodium, le chlorure de potassium, le chlorure de magnésium, le chlorure de calcium, le bromure de lithium, le bromure de sodium, le bromure de potassium ou le bromure de magnésium.

13. Procédé selon la revendication 1, **caractérisé en ce que** dans la deuxième étape on utilise comme source de chlorure ou bromure supplémentaire l'acide chlorhydrique ou bromhydrique.
